(19) **European Patent Office** — Europäisches Patentamt / European Patent Office / Office européen des brevets

(11) **EP 1 238 590 A1**

(12) # EUROPEAN PATENT APPLICATION

(43) Date of publication:
**11.09.2002 Bulletin 2002/37**

(21) Application number: **01200881.9**

(22) Date of filing: **09.03.2001**

(51) Int Cl.$^7$: **A23D 7/00**, A23D 9/007,
A23L 1/24, A23G 9/02,
C07J 13/00

(84) Designated Contracting States:
**AT BE CH CY DE DK ES FI FR GB GR IE IT LI LU
MC NL PT SE TR**
Designated Extension States:
**AL LT LV MK RO SI**

(71) Applicant: **UNILEVER N.V.
3013 AL Rotterdam (NL)**

(72) Inventors:
• **Beindorf, Christiaan
c/o Unilever Research & Dev.
3133 AT Vlaardingen (NL)**

• **Husken, Henk c/o Unilever Research & Dev.
3133 AT Vlaardingen (NL)**
• **Zijp, Itske, M. c/o Unilever Research & Dev.
3133 AT Vlaardingen (NL)**

(74) Representative: **Kleiborn, Paul Erik et al
Unilever N.V., Patent Department,
P.O. Box 137
3130 AC Vlaardingen (NL)**

(54) **Fat based food product**

(57)    A fat based food product is described which comprises an amount of a deodorized Guggul ingredient. Preferably the deodorized Guggel ingredient is decoloured.

EP 1 238 590 A1

## Description

### Field of the invention

The present invention concerns a fat based food product comprising components which have a blood cholesterol lowering effect.

### Background to the invention

A fat based food product comprising components which have a blood cholesterol lowering effect is known.

For instance, US 3,751,569 suggests the use of esters of monocarboxylic acid and plant sterols in dietary oils for reducing the cholesterol level.

WO 96/38047 (Unilever) describes the addition of specific levels of phytosterols to fat based food products, the phytosterols being defined as to include phytosterols fatty acid esters. The phytosterol comprising fat based food products have a blood cholesterol lowering effect.

Other compounds having blood cholesterol lowering effect that have been used in fat based food products are conjugated linoleic acid, see for instance EP-A-954975.

Though many compounds are reported to have blood cholesterol lowering effect, some of which are available as medicines, most of these compounds are not used as additives to fat based food products.

One group of compounds that have a reported blood cholesterol lowering effect are derived from the plant *Commiphora mukul*.

The *Commiphora mukul* is a small tree of the Burseraceae family, endemic in the Indian peninsula. Since ancient times a medicine is derived from this tree, by etching the trunk of the tree in winter times. The etched trunk exudes a gum-resin which is known as Guggulu. The ethyl acetate extract of Guggulu is known as Guggulipid. Guggulipid and other Guggulu ingredients are reported to have a blood chlolesterol lowering effect. Guggulipid contains C21 and C27 steroids, with the major constituents being Z-Guggulsterone and E-Guggulsterone. These sterones are believed to be the active substances in Guggulipid.

From Sharma K., Jour. Res. Ind. Med. 1976; 11(2), 132-134 it is known that Guggulu has a cholesterol lowering activity.

In a human study it was shown that 4 g/d Guggulu taken for 4 weeks gave a total cholesterol decrease of around 15%. From Singh R.P. et al, Int J Pharmacog 1993;30(2),147-160, it is known that Guggulipid has a cholesterol lowering activity. A human study was conducted with a 12 week diet stabilization period, in which total cholesterol was lowered 12% and subsequently a 24 week period in which 2.4 g/day Guggulipid was taken which gave a further 11% decrease in total cholesterol.

Fraction A was reported to have a cholesterol lowering activity in human study, Kotiyal, J.P. et al, J Res Ayur Siddha 1980; 335-344. The decrease in serum cholesterol reported is 9% in a 4 weeks study using 1.5g/day of Fraction A.

Based on these and other studies in the literature we herein assume that an intake of 4-6 g/day Guggulu, 1.5-2.5 g/day Guggulipid, 1.5 g/day fraction A or a corresponding amount of 60-120 mg/day of Z- and E- Guggulsterones, is effective in lowering blood cholesterol.

US 5,273,747 discloses a process for the preparation of a product consisting essentially of Guggulsterones and Guggulsterols and therapeutic use of such a product.

A commercially available tablet comprising Guggul extract is known as Cholestame (www.iherb.com/iherb/chlolestame.html). The tablets are said to promote the normal cholesterol metabolism.

EP-A-0997149 discloses compositions comprising guggul extract and phosphate salts and a weight control product comprising the compositions. The compositions are also used as a medicament for reducing plasma cholesterol levels in a mammal. The weight control product may be administered in the form of a capsule, tablet, powder, food bar, soft gel capsule and the like. It does not disclose fat based food products.

Now, we have found that organoleptically acceptable fat based food products cannot be prepared from Guggulu, Guggulipid, Fraction A or other Guggul ingredients as such. Incorporation of these products in fat based food products, in an amount sufficient to get a cholesterol lowering effect, was found to lead to an unacceptable off-flavour of the prepared food products, such that they could not be used as food products.

### Summary of the invention

An object of the invention is to provide novel fat based food products having a blood cholesterol lowering effect. Another object of the invention is to provide fat based food products having an improved blood cholesterol lowering effect, compared to known fat based food products. Another object is to provide fat based food products having a good taste and flavour.

These objects are attained according to the invention in that the food product comprises an amount of a deodorized Guggul ingredient.

**Detailed description of the invention**

Fat based food products are food products (partially) based on fat and regarded by the consumer, as 'fatty type of products'. Examples are yellow fat spreads (containing vegetable fat and/or animal fat such as butterfat), dressings, coffee-creamer, shortenings, cooking and frying oils, fillings and toppings, ice-cream and the like. These products in most cases comprise a particular amount of fat. In some cases, however, products are still regarded as 'fatty type of products', despite a replacement of part or even all the fat by fat replacers. Fat based food products in which the fat is partially or completely replaced by fat replacers are also covered by the term fat based food products of this invention.

The food products as such are common products in the western world, and are used by consumers on a daily basis in amounts different for each individual.

The invention is in particular very suitable for yellow fat spreads, dressings, cheese, shortenings, cooking and frying oils and ice cream, with a preference for yellow fat spreads, mayonnaise, dressings, shortenings, cooking and frying oils. On the basis of habits of the consumer in the western world, the invention is preferred to concern particular for yellow fat spreads (including margarines, butter and low fat spreads) and dressings. Yellow fat spreads, for this invention, can comprise 0 (zero) to 90% fat (usually 5-80%). Dressings can comprise 0 to 85% fatty (usually 5-80%), shortenings, cooking and frying oil more than 95% fat. In a preferred embodiment, the food product is a yellow fat spread comprising 40 to 80% fat.

The fat that is applied in these fat based food products can be any fat, such as dairy fat and/or vegetable fat. However, if fat is present, for health reasons the use of one or more vegetable fat sources is preferred. In particular, the use of liquid fats is preferred. The fat can be one single fat or a blend. The use of fat compositions comprising a considerable amount of PUFA rich triglycerides in addition to the use of the sterol/sterol ester mixture is in particular considered highly beneficial. For example, oils of sunflower, safflower, rapeseed, linseed, linola and/or soybean can be used in a preferred embodiment. Also the fat compositions mentioned in Netherlands patent documents no. Nl 143115, Nl 178559, Nl 155436, Nl 149687, Nl 155177, European patent documents EP 41303, EP 209176, EP 249282, and EP 470658 are highly suitable.

If a fat blend is used, it is preferred that it comprises at least 30%, and more preferred at least 45% of polyunsaturated fatty acids, based on the total weight amount of the fat in the fat based food product. So, a strong effect on the cholesterol lowering effect is obtained if use is made of an optimal ratio of Guggul ingredients as set forth in this application in a food product in which a fat blend comprising at least 30 wt.% of PUFA rich triglycerides is used.

In a particular embodiment, the fatty acid mixture contains a high amount (>35%, preferably >45%, further preferred >60%) of polyunsaturated fatty acids (PUFA).

The deodorized Guggul ingredient according to the invention includes any Guggulsterone containing material that has been subjected to a deodorization process.

As feed to the deodorization process, any Guggulsterone containing material may be used, such as Guggulu, Guggulipid, Fraction A,$CO_2$-extract of Guggulu or any comparable product that contains an amount of Guggulsterones.

The Guggulsterone containing material is preferably in a liquid state during deodorization. A suitable solvent, such as oil, may have to be added to get the prefered liquid Guggulsterone containing material.

The deodorization process may be conducted by stripping the Guggulsterone containing material with an inert gas, such as steam or nitrogen. The deodorization conditions, such as pressure, temperature and inert gas flow and reaction time, are chosen such that no substantial evaporation of Z-Guggulsterone and E-Guggulsterone occurs, but on the other hand a substantial part of the flavour components in the Guggulsterone containing material is carried away by the stripping gas.

Preferred ranges for the deodorization temperature are 50-180°C, more preferably 100-140°C, most preferably around 120°C. Too high temperatures may cause removal of favourable components. The deodorization pressure may be any pressure, but preferably the pressure is sub-atmospheric, in order to achieve a large volume of deodorization gas. When steam is used as inert gas, the pressure is preferable from 0.05-100 Pa, more preferably from 0.1-0.5, most preferably about 0.2 Pa. When nitrogen is used as a stripping gas, higher pressures may advantageously be used.

The deodorization may be conducted in a deodorization tower, preferably a conventional deorization tower, such as used in the deodorization of oils and fats. See for instance O.L. Brekke in Handbook of Soy Oil Processing, American Soybean Association, 1980, pages 173 ff.

Preferably the Guggul ingredient is decoloured. Advantageously the decoloration process is conducted by filtration, especially preferred by ultrafiltration or membrane filtration. See for a description of membrane filtration WO 98/33199.

The deodorized and optionally decolourized Guggul ingredient is used as ingredient in the fat based food products according to the invention and may be added in any step during the preparation of the food product. Suitably, the deodorized Guggul ingredient may be added to and dissolved in the fat prior to combining with the aqueous phase of the product to be prepared.

The amount of deodorized Guggul ingredient in the food product should be such that a blood cholesterol lowering effect is achieved. Preferably the amount is such that the amount of Guggulsterones in the food product is 40-400 mg/weight of an average daily serving of the food product, more preferably 40-200, most preferbaly 60-120 mg/weight of an average daily serving of the food product.

The skilled person will be able to adjust the amount of deodorized Guggul ingredient in the food product to get the above effect. The percentages will depend on the type of food product, since the food products are used in different serving sizes. Moreover the pattern in a food product is consumed (servings per day and distribution over days) is dependent on the food product. Furthermore differences between countries in eating patterns occur.

Average daily servings may be derived e.g. from data published by governmental institutes. An example of a study giving average daily servings is: Gregory et.al, The Dietary and Nutritional Survey of British adults: A survey carried out by the Social Division of OPCS with dietary and nutritional evaluations by the Ministry of Agriculture, Fisheries and Food and the Departmnent of Health, United Kingdom, 1990.

For example, for a margarine, assuming that the average daily serving is 20 grams, the skilled person can make the following calculation. When a daily amount of 60-120 mg/day Guggulsterones is desired, in the margarine, the amount of Guggulsterones should be 3-6 mg/g, or 3000-6000 mg/kg. For other fat based products according to the invention an analogous calculations can be made.

**Examples**

Determination of Yellowness index

The yellowness index is determined from samples at 15 °C, on a Tricolor LFM3 Colorimeter (Dr Lange). The Yellowness Index is calculated from the tristimulus colour (in X, Y and Z) of the sample, referred to the standard illuminant C (white plate) and the 2 °C observer according to the formula:

$$Y.I. = \frac{1.28 \times X - 1.06 \times Z}{Y} \times 100$$

Determination of colour (L*a*b)

When colours are classified, they can be broken down into the three primary elements. One is the Hue (colour) the other is Value (brightness) and the third is Chroma (Saturation like vivid colours or dull colours).

To enable anyone to tell anyone else exactly what colour they are talking about a common numerical code is used. This numerical code used is L*a*b*. When a colour is expressed in this system, Value becomes L*, while Hue and Hue are expressed as a* and b* respectively. The L*a*b* was measured of different time samples during fermentation. The supernatant of the samples was filtered sterilized with a milipore 0.22µm filter. Of the clear liquid L*a*b* was measured with a UV 1601 spectofotometer of Shimatzu.

**Example 1**

A. Deodorization of Guggulipid

To 50 g Guggulipid (ethyl-acetate extract of Guggulu)containing 4.9 wt.% E-Guggulsterone and 2.3 wt.% Z-Guggulsterone, 450 g refined Sunflower oil (ex. Chempri b.v. Oleochemicals) was added. The mixture was stirred and heated to 60°C. The obtained Guggulipid solution was filtrated at 60°C over a Seitz K100 filter to obtain a clear solution. 399.4 g of the Guggulipid solution was put in a 1 liter deodorizer. The deodorizer was slowly evacuated to 0,2 Pa (2 mBar). The Guggulipid solution was slowly heated to 120°C. A steam flow of 1% water/kg oil*h was applied and the Guggullips solution was deodorized for 2.5 hours. A deodorized Guggulipid solution resulted. An analysis with gaschromatography showed a concentration of E-Guggulsterone of 0.3 wt.% en Z-Guggulsterone of 0.1 wt.%.

B. Preparation of a margarine

The following margarine composition was prepared:
Fat phase (82 wt.% of the margarine):

0.18 wt.% Lecithin (Bolec MT, Unimills, Zwijndrecht, Netherlands)
0.1 wt.% lecithin (Cetinol Unimills, Zwijndrecht, Netherlands)
1.95 wt.% hydrogenated rape oil
79.62 wt.% of the diluted deodorized Guggulipid solution as described previously.
Water phase (18 wt.% of the margarine)
100 wt.% Demineralized water

The flavour of the margarine was acceptable.The margarine contained 3200 mg/ kg Guggulsterones (total of Z- and E-guggulsterone).

## Example A (comparative example)

Example A was executed as Example 1. Instead of diluted deodorized Guggulipid solution, sunflower oil in which 10 wt.% Gugullipid was dissolved, was used.

The margarine has a strong off-flavour and was organoleptically unacceptable for use as a margarine.

## Example 2

An Amicon dead-end cell containing a YM2 membrane was filled with 148.9g of a 3% guggulipid solution in ethanol. Filtration was performed at 22°C and 5.7 bar (nitrogen). The starting solution had a brown-red colour with a typical guggulipid odour. When 98.0g of permeate was collected, the filtration was stopped. The permeate had a light yellow-brown colour. The colour of the permeate and the starting solution are reported in table 1. After evaporation of the ethanol under vacuum in a rotavapor, 2.72g of guggulipid with a light yellow-brown appearance was obtained. Guggulipid haa a dark brownish black colour.

Table 1

| Colour of guggulipid solutions in ethanol | | | |
|---|---|---|---|
| 3% Guggulipid solution in EtOH | L*a*b | | |
| | L | a | b |
| Start solution | 75.1 | +7.8 | 92.0 |
| Permeate | 91.0 | -11.2 | 55.1 |

The decoloured guggulipid was diluted 10-fold with sunflower oil and deodorized in the same way as was described in example 1. Subsequently the decolourized and deodorized guggulipid solution was used to prepare a margarine. The margarine had no off-flavour and an acceptable light yellow colour compared to the comparative margarine (example A) which had an off-flavour and a light brown colour. Colours of the margarines are reported in table 2.

Table 2:

| Yellowness index and L*a*b values of margarines containing guggulipid | | | | |
|---|---|---|---|---|
| Margarine with Guggulipid Ingredient | Yellowness index | L | a | b |
| Decolourized and deodorized guggulipid | 43.8 | 80.8 | -2.3 | 23.4 |
| Guggulipid | 69.7 | 71.9 | 1.1 | 34.9 |

## Claims

1.  Fat based food product, **characterised in that** the food product comprises an amount of a deodorized Guggul ingredient.

2.  Fat based food product according to claim 1, wherein the amount of a deodorized Guggul ingredient is such that the amount of Guggulsterones in the food product is 40-400 mg/weight of an average daily serving of the food product.

3. Fat based food product according to claim 2, wherein the amount of a deodorized Guggul ingredient is 60-120 mg/ weight of an average daily serving of the food product.

4. Fat based food product according any one of claims 1-3, wherein the deodorized Guggel ingredient is decoloured.

5. Fat based food product according to any of claims 1-4, wherein the fat used in the product is a fat comprising at least 30 wt.%, and preferably at least 45 wt.% of PUFA rich triglycerides, calculated on the total weight of the fat present in the product.

6. Fat based food product according to any of claims 1-3 selected from the group of yellow fat spreads, mayonnaise, dressings, shortenings, cooking and frying oils and ice-cream.

7. Process for the preparation of a fat based food product comprising an amount of one or more guggul sterones, in which Guggulipid is subjected to deodorization and the deodorized Guggulipid is used in the preparation of the fat based food product.

8. Process according to claim 4, wherein the Guggulipid is subjected decolorization, prior to, during or after deodorization and the decolorized Guggulipid is used in the preparation of the fat based food product.

9. Process according to claim 5, wherein the decolorization is conducted through membrane filtration of the Guggulipid.

**European Patent Office**

**EUROPEAN SEARCH REPORT**

Application Number

EP 01 20 0881

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | PATENT ABSTRACTS OF JAPAN vol. 1996, no. 06, 28 June 1996 (1996-06-28) & JP 08 033464 A (SEIKOSHA:KK), 6 February 1996 (1996-02-06) * abstract * | 1 | A23D7/00 A23D9/007 A23L1/24 A23G9/02 C07J13/00 |
| Y | WO 97 39355 A (PHARMAPRINT INC ;UNIV SOUTHERN CALIFORNIA (US); KHWAJA TASNEEM A () 23 October 1997 (1997-10-23) * page 7, line 18 - line 36 * * page 24, line 20 - line 37 * * page 25, line 19 - line 26 * * claims 11,66,78 * | 1 | |
| A | WO 01 05356 A (NATROL INC) 25 January 2001 (2001-01-25) * page 16, line 13 - line 15 * * claims 1,2,6,10 * | 1 | |
| D,A | EP 0 997 149 A (PROLAB NUTRITION INC) 3 May 2000 (2000-05-03) * page 5, line 7 - line 10 * * claim 1 * | 1 | **TECHNICAL FIELDS SEARCHED (Int.Cl.7)** A23D A23L A23G C07J |
| A | BEG M ET AL: "A study of effect of #guggulsterone# on hyperlipidemia of secondary glomerulopathy" INDIAN JOURNAL OF PHYSIOLOGY AND PHARMACOLOGY,IN,ASSOCIATION OF PHYSIOLOGISTS AND PHARMACOLOGISTS, vol. 40, no. 3, July 1996 (1996-07), pages 237-240, XP002118411 ISSN: 0019-5499 * page 238, column 1, paragraph 2 * | 1 | |

The present search report has been drawn up for all claims

| Place of search | Date of completion of the search | Examiner |
|---|---|---|
| THE HAGUE | 1 August 2001 | Dekeirel, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**          EP 01 20 0881

This annex lists the patent family members relating to the patent documents cited in the above-mentioned European search report.
The members are as contained in the European Patent Office EDP file on
The European Patent Office is in no way liable for these particulars which are merely given for the purpose of information.

01-08-2001

| Patent document cited in search report | | Publication date | Patent family member(s) | | Publication date |
|---|---|---|---|---|---|
| JP 08033464 | A | 06-02-1996 | NONE | | |
| WO 9739355 | A | 23-10-1997 | AU | 716155 B | 17-02-2000 |
| | | | AU | 2813197 A | 07-11-1997 |
| | | | CA | 2252426 A | 23-10-1997 |
| | | | CN | 1239547 A | 22-12-1999 |
| | | | EP | 0900375 A | 10-03-1999 |
| | | | JP | 2000512621 T | 26-09-2000 |
| | | | US | 6039950 A | 21-03-2000 |
| WO 0105356 | A | 25-01-2001 | AU | 6221500 A | 05-02-2001 |
| EP 0997149 | A | 03-05-2000 | US | 6113949 A | 05-09-2000 |
| | | | GB | 2343115 A | 03-05-2000 |

EPO FORM P0459

For more details about this annex : see Official Journal of the European Patent Office, No. 12/82